# EUROPEAN PATENT SPECIFICATION

(11) **EP 0 840 618 B1**
(45) Date of publication and mention of the grant of the patent: **23.04.2003**
(21) Application number: 96915808.8
(22) Date of filing: 16.05.1996
(51) Int. Cl.: A61K 39/395, C07K 16/28

(54) **INDUCTION OF IMMUNOLOGICAL TOLERANCE BY THE USE OF NON-DEPLETING ANTI-CD4 ANTIBODIES**
INDUKTION VON IMMUNOLOGISCHER TOLERANZ UNTER VERWENDUNG VON NICHT-ZELLMENGEVERRINGERNDEN ANTI-CD4-ANTIKOEPERN
DECLENCHEMENT DE TOLERANCE IMMUNOLOGIQUE AU MOYEN D'ANTICORPS ANTI-CD4 NE PROVOQUANT PAS DE DEPLETION

(30) Priority: 18.05.1995 US 443739
(43) Date of publication of application: 13.05.1998
(60) Divisional application: 01202886.6
(73) Proprietor: Ortho-McNeil Pharmaceutical, Inc., Raritan, NJ 08869 (US)
(72) Inventor: KNOWLES, Robert, W., New York, NY 10021 (US); CAVENDER, Druie, E., Flemington, NJ 08822 (US); THOMAS, Judith, M., Birmingham, AL 35242 (US)
(74) Representative: Mercer, Christopher Paul
(86) International application number: US9606912
(87) International publication number: WO96036359

(56) References cited:
- EP-A- 0 240 344
- WO-A-91/09966
- WO-A-92/05274
- TRANSPLANTATION PROCEEDINGS, vol. 25, no. 1, 1993, pages 784-785, XP002009900 DELMONICO, F.L. ET AL.: "Immunosuppression of cynomolgus renal allograft recipients with humanized OKT4A monoclonal antibodies"
- TRANSPLANTATION, vol. 57, 1994, pages 788-793, XP002009901 POWELSON, J.A. ET AL.: "CDR-grafted OKT4A monoclonal antibody in cynomolgus renal allograft recipients" cited in the application
- TRANSPLANTATION PROCEEDINGS, vol. 25, no. 1, 1993, pages 794-795, XP002009902 WEE, S. ET AL.: "Potent CD8+ CTLs detected in anti-CD4 (OKT4A) mab immunosuppressed cynomolgus recipients with prolonged allograft survival"
- SEMINARS IN IMMUNOLOGY, vol. 2, 1990, pages 377-387, XP002009903 COBBOLD, S.P. ET AL.: "reprogramming the immune system for tolerance with monoclonal antibodies"
- TRANSPLANTATION, vol. 54, 1992, pages 483-490, XP002009904 DARBY, C.R. ET AL.: "Evidence that long-term carciac allograft survival induced by anti-CD4 monoclonal antibody does not require depletion of CD4+ T cells" cited in the application
- Biol. abstracts, vol. 47, abs. No. 160914 & 9th, Int Congress of Immunology, San Francisco, July 23 - 29, 1995
- Rebellato et al, Transplant. Proc. vol. 25, 1993, pp. 598 - 599
- Thoams et al, Transplantation vol. 59, 1995, pp. 245 - 255

## Description

### BACKGROUND OF THE INVENTION

The present invention relates to the use of non-depleting anti CD-4 antibodies in combination with donor bone marrow to inhibit allograft rejection.

The immunosuppressive effects of antibodies against T-lymphocytes are well known. Various monoclonal antibodies which have been shown to induce immunosuppression may be characterized as either depleting antibodies or as non- depleting antibodies. Depleting antibodies are those which cause depletion of cells carrying their target antigen in the recipient. Non-depleting antibodies are those which do not cause depletion of cells carrying their target antigen.

Tolerance induction has been observed in the mouse cardiac allograft model with non-depleting anti-CD4 mAb as described in Darby et al., Transplantation, 54:483-490 September 1992. Prolongation of kidney allograft survival in rhesus monkeys with murine OKT4A is described in Jonker et al., Transplantation, 39:247, 1985. Rebellato et al, Transplantation Proc 25: 598-99 (1993) describe the administration of a mixture of monoclonal antibodies (anti-Cd2, anti-CD4 and anti-CD8) to rhesus monkey allograft recipients. In WO92/05274, Gorman et al disclose four CDR-grafted anti-CD4 antibodies and propose that these antibodies could be used to induce tolerance to an antigen, an organ graft or a bone marrow transplant. Prolongation of kidney allograft survival in monkeys with a non-depleting antibody, humanized OKT4A is described in Powelson, et al., Transplantation 57:788-793, March 1994. Although Powelson describes prolongation of kidney allograft in primates using a non-depleting anti-CD4 antibody, induction of long term immunological tolerance has not been accomplished in primates. Furthermore, it has been established that tolerance induction in mice is relatively easy to accomplish, and in no way indicates that such tolerance will be possible in higher mammals.

The infusion of donor bone marrow in monkeys treated with rabbit anti thymocyte globulin promotes graft tolerance without chronic immunosuppression (Thomas et al., Transplantation 51:198, 1991). Retrospective analysis of long term graft survival in this model has revealed that partial matching between the donor and recipient animals, (one shared allele at the DRB1 major histocompatability gene), increased long term allograft survival following treatment with rabbit anti-thymocyte globulin and donor bone marrow (Thomas et al., Transplantation 59:245 255, 1995).

### SUMMARY OF THE INVENTION

The present invention also provides a method of inhibiting allograft rejections without depleting the patient's T-cell population. Allograft rejection is inhibited by administering to the patient, a non-depleting, humanized anti-CD4 antibody in combination with bone marrow cells from the graft donor.

### BRIEF DESCRIPTION OF THE FIGURES

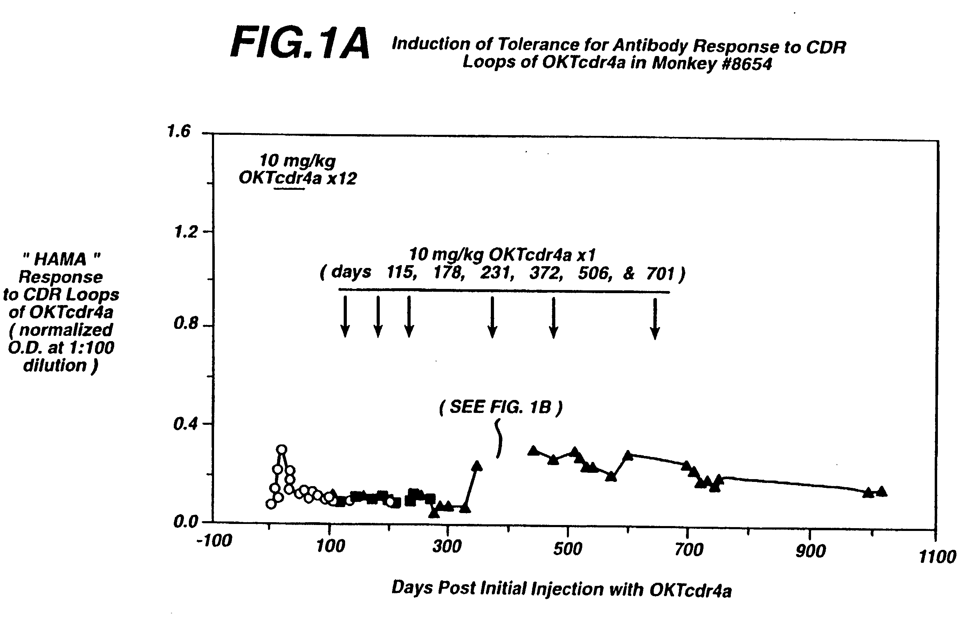

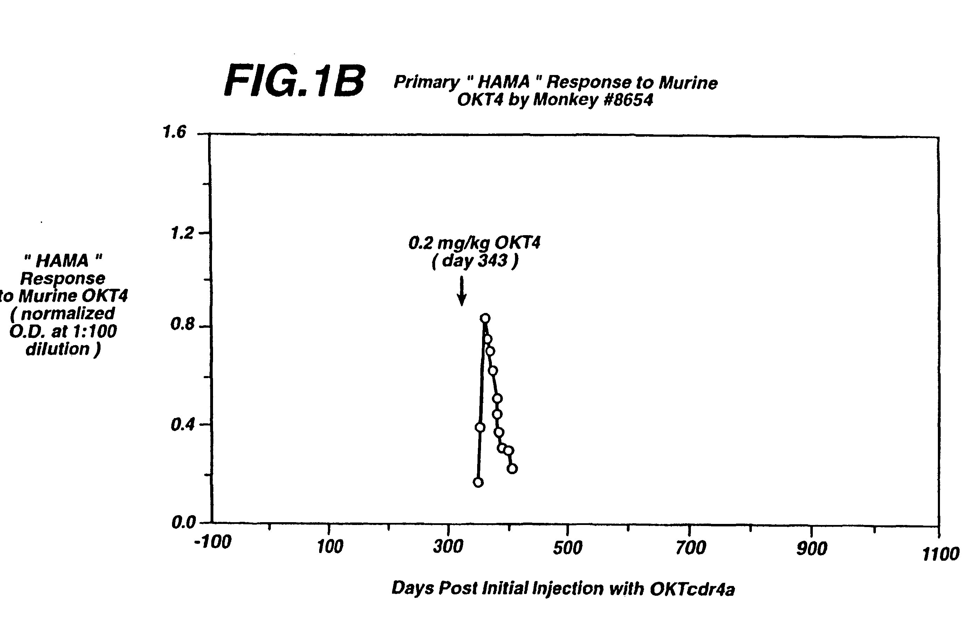

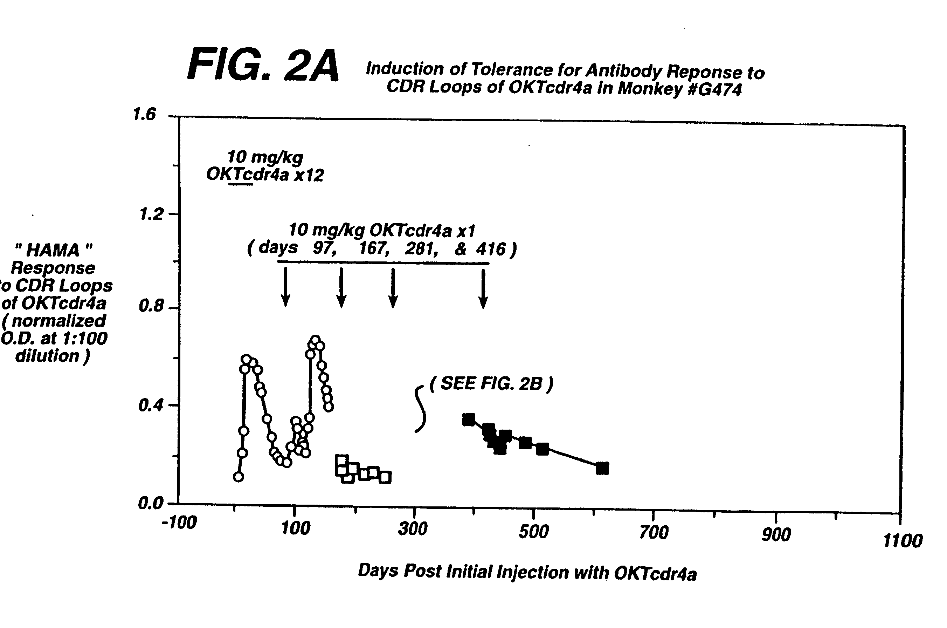

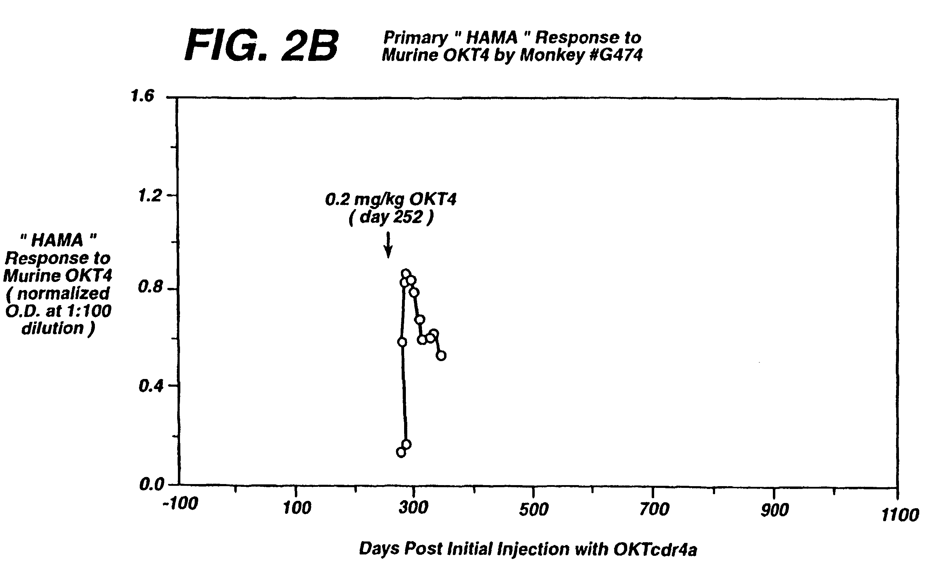

Brief description of Figures 1A, 1B, 2A, and 2B.
Anti-antibody responses in the two monkeys injected with 10 mg/kg/day of OKTcdr4a for 12 days are shown. An ELISA was designed to detect monkey antibodies that were capable of binding to solid-phase murine OKT4A, which shares its CDR loops in common with OKTcdr4a. The plates could not be coated with OKTcdr4a since any reagent that could detect monkey HAMA would cross-react with OKTcdr4a. Monkeys injected with OKTcdr4a were not expected to produce antibodies to the humanized portions of the molecule due to the high sequence homology of monkey and human immunoglobulins.

Microtiter plates were coated with murine OKT4A. Following the blocking of non-specific adsorption sites with BSA, monkey samples at dilutions >1:100 were added. Binding of monkey HAMA to the plates was detected with a HRPO-conjugated goat anti-monkey IgG (H+L) reagent. A substrate solution containing H2O2 and orthophenylenediamine was then used to develop the reaction.

O.D. readings were normalized to the O.D. reading obtained with a slightly subsaturating dilution of a secondary HAMA-containing sample obtained from another monkey. A 1:100 dilution of a pool of plasma samples from eleven non-injected monkeys was also run on each plate as a negative control. Titers of HAMA were defined as the dilution at which the normalized O.D. value of the peak sample from an injected animal was equal to twice the normalized O.D. reading of the negative control pool. Inhibition experiments on positive samples with soluble mOKT4A or OKTcdr4a demonstrated that the monkey HAMAs were inhibited equally well with either mAb (data not shown), confirming that the murine and humanized antibodies share antigenic epitopes within the CDR loops regions.

Plasma samples were tested by ELISA for the presence of HAMA to OKT4, using the same protocol as the anti-CDR-loops assay described above, except that the samples were tested on plates coated with either mOKT4 or mOKT4A. Since nearly identical O.D. readings were obtained on the two types of plates, the data are presented as the normalized O.D. values from the mOKT4A-coated plate since the raw O.D. readings on that plate could be normalized to the O.D. valueobtained with the positive control run on that plate.

### DETAILED DESCRIPTION OF THE INVENTION

Immunological tolerance is defined as immunological unresponsiveness to challenge with the antigen to which tolerance has been induced. Tolerance is demonstrated when a subject is unresponsive subsequent to challenge with the tolerance-inducing antigen.

The cdr-grafted anti-CD4 antibody designated OKT cdr4a used the present invention is described in International Application Number PCT/GB90/02015, published as International Publication Number WO 91/09966, the entire contents of which is hereby incorpated by reference.

### Experiment 1: Induction of Antigen-specific Tolerance by OKTcdr4a in Cynomolgus Monkeys (not part of the invention as covered by the present claims)

Tolerance measured by the HAMA response to OKTcdr4a

Since the HAMA response to the injected OKTcdr4a is easily measured, this immune response was used to determine whether some treatment protocols can induce immunological tolerance to OKTcdr4a itself and therefore might be useful clinically to induce tolerance to allografts and autoantigens.

Cynomolgus monkeys were injected with 0.2, 1, or 10 mg/kg/day ofOKTcdr4a, either on a single day or on 12 consecutive days. Although every monkey (n = 13) made an anti-antibody response to the CDR loops of OKTcdr4a, the two monkeys injected with 10 mg/kg/day of OKTcdr4a for 12 days (the highest dose tested) made the weakest and most transient anti-antibody responses (titers less than 1:1,500, which declined to background by day 40) (Table 1). One major difference observed in these animals was the length of time that the CD4 molecules on the peripheral blood CD4+ T cells remained saturated with OKTcdr4a. In this study with cynomolgus monkeys the critical time point was approximately 30-60 days post inital treatment.

Since immunological tolerance is a long term goal of anti-CD4 therapy, based on the early success obtained in rodent model systems (Shizuru et al.6, Cobbold et al.7) two animals that were treated with 10 mg/kg/day of OKTcdr4a for 12 days were further studied to determine whether antigen specific immunological tolerance in the human anti-mouse antibody (HAMA) response to OKTcdr4a had been induced. After the plasma levels of OKTcdr4a in these two monkeys had declined to <1 _g/ml, each was challenged with a single 10 mg/kg dose of OKTcdr4a. One animal made no anti-antibody response at all, while the second monkey made a weak, transient response that resembled its primary response. The two monkeys were then challenged 5-6 additional times with OKTcdr4a over a period of 600-700 days, and no significant anti-antibody responses were observed (Figures 1A and 2A). In contrast, three of the remaining 11 monkeys were similarly challenged with a single 10 mg/kg dose of OKTcdr4a, and all made typical strong secondary anti-antibody responses (mean peak titer of 1:157,000). The two animals treated with 10 mg/kg/day of OKTcdr4a for 12 days appeared to be specifically unresponsive to OKTcdr4a since they made normal antibody responses to an unrelated protein antigen, murine OKT4, (Figures 1B and 2B) and suffered no obvious immunodeficiency.

These results support the conclusion that the 10 mg/kg/day for 12 days protocol can induce a state of tolerance with regard to the HAMA response. Since both the murine and OKTcdr4a antibodies have higher affinities for human CD4 than for monkey CD4, lower doses and shorter dosing schedules may be sufficient to induce a similar state of tolerance in human patients.

### Experiment 2: Immunosuppression of Renal Allograft Rejection with OKTcdr4A in Cynomolgus Monkeys

In a previous study with OKTcdr4a in a non-human primate model of allograft rejection, the immunosuppressive activity of OKTcdr4a. This resulted in extended allograft survival times averaging 35 days (n=3), compared to 9 days in untreated recipients (n=4). However, a strong anti-antibody response was observed in each animal tested, associated with the loss of CD4 saturation, and long term immunological tolerance was not acheived in this model (Powelson et al. 1994). Long term immunological tolerance induction has also been demonstrated in a non-human primate model of renal allograft rejection with a pllyclonal rabbit anti-monkey thymocyte antiserum that depletes CD4= T cells from the peripheral blood, in combination with the administration of an HLA-DR (-/dim) fractioned cell population from donor bone marrow. (Thomas et al, Transplantation 51:198, 1991). With a protocol based on the previous study with rabbit anti-monkey thymocyte antiserum and donor bone marrow in the rhesus monkeu model, a study was designed to test whether OKTcdr4a could be substituted for the rabbit antiserum.

OKTcdr4a was administered intravenously to 13 rhesus monkey renal allograft recipients at a dose of 10 mg/kg/day for 21 days, beginning on the day of transplant, following partial MHC matching between the donor and recipient animals, (one shared allele at the DRB1 gene locus), as described previously (Thomas et al., 59:245-255, 1995). For 7 allograft recipients, donor bone marrow cells (Dr -/low) were prepared from bone marrow isolated from the allograft donor, as described previously (Thomas et al, Transplantation 51:198, 1991).

Four protocols were used, as shown in Table 2. (1) OKTcdr4a was used as the only immunosuppressive therapeutic (21 days, beginning on the day of transplant); (2) OKTcdr4a (21 days) was administered with the addition of donor bone marrow, on day 8 or day 9 post transplantation. (3) OKTcdr4a (21 days) was administered with the addition of cyclosporine 20 mg/kg p.o. on days 0-7 post transplant; (4) OKTcdr4a (21 days) was administered with the addition of cyclosporine 20 mg/kg orally on days 0-7 post transplant plus donor marrow, administered on day 8 or day 9 post transplantation.

OKTcdr4a treatment facilitated graft survival over 100 days in 5 out of the 13 recipients. Since long term graft survival times over 100 days were achieved in 3 out of 3 recipients using OKTcdr4a with donor bone marrow, OKTcdr4a treatment may establish a favorable milieu for tolerance induction with donor bone marrow cells.

Long term allograft survival also correlated with weak anti-antibody responses. Four of the 13 animals were strong responders with peak anti-antibody titers above 1:200,000 during dosing, resulting in reduced plasma levels of OKTcdr4a and reduced CD4 receptor occupancy. Three of these strong responders were cyclosporine-treated recipients. The anti-antibody response status also correlated with the anti-donor antibody response. None of the six low anti-antibody responders made detectable anti-donor antibody responses before 100 days, whereas three of the four high anti-antibody responders had positive anti-donor responses.

These studies suggest long term allograft survival and suppression of anti-donor antibodies can be achieved, particularly after donor bone marrow infusion, by prolonged administration of OKTcdr4a to block CD4 receptors, provided the anti-antibody response is suppressed. This OKTcdr4a therapy appears to induce tolerance to OKTcdr4a, a process that may have been inhibited by a short course of cyclosporine. Concomitant suppression of anti-donor responses in low anti-antibody responders likely reflects inhibition of CD4-dependent T cell help to B cells by maintaining saturation of CD4 receptors.

**Table 1.**

| Anti-antibody responses to the "CDR loops" of OKTcdr4a and CD4 saturation in monkeys treated with OKTcdr4a. | | | | |
|---|---|---|---|---|
| Monkey ID | Protocol dose(mg/kg/d) x days | Duration of CD4 Saturation days >80% a | Primary Response 1 / tiler | Secondary Respnse 1 / titer |
| 2124 | 0.2 x 1d | 0-1 | 10,000 | |
| 2138 | 0.2 x 1d | 0 | 6,000 | |
| | | | | |
| 2102 | 1 x 1d | 0-1 | 7,000 | |
| 2116 | 1 x 1d | 2-3 | 18,000 | |
| | | | | |
| 8521 | 10 x 1d | 8-11 | 6,000 | 70,000 |
| 2141 | 10 x 1d | 11-18 | 12,000 | |
| 2111 | 10 x 1d | 12-14 | 26,000 | |
| | 300,000 | | | |
| | | | | |
| 8912 | 0.2 x 12d | 11-12 | 8,000 | 100,000 |
| F798 | 0.2 x 12d | 12-14 | 3,000 | |
| | | | | |
| 8920 | 1 x 12d | 11-12 | 30,000 | |
| G551 | 1 x 12d | 11-12 | 85,000 | |
| | | | | |
| G493 | 10 x 6d | 20-27 | 21,000 | |
| | | | | |
| 8654 | 10 x 12d | 53-57 | 400 | 0 |
| G474 | 10 x 12d | 34-41 | 1,500 | 750 |

| | | | | |
|---|---|---|---|---|
| a Duration of saturation is indicated by the last day a sample was found to be >80% saturation and the first day a sample was found to be <80% saturation. | | | | |

**Table 2.**

| Graft survival, anti-antibody responses to the "CDR loops" antigen of OKTcdr4a and CD4 saturation in rhesus monkey renal allograft recipients treated with OKTcdr4a alone or in combination with cyclosporine A, and / or donor bone morrow. | | | | | |
|---|---|---|---|---|---|
| Monkey ID# | Protocol (mg/kg/d x d) ±DBM±CspA | Graft Survival days | Duration of Plasma >100µg/ml | Duration of CD4 Satur. >80% | Anti-mAb Response 1 / titer |
| 8R2 | 10 x 21d | 170 | 84 | 100 | 1,000 |
| 91B078 | 10 x 21d | 23 | 16 | 15 | 700,000 |
| FOC | 10 x 21d | 54 | 54 | 54@ | >100 |
| | | | | | |
| 91B089 | 10 x 21d +DBM | >248 | 66 | 70 | 1,000 |
| 91B116 | 10 x 21d +DBM | 110 | 65 | 98 | 100 |
| F3F | 10 x 21d +DBM | >109 | >56 | 84 | 7,000 |
| | | | | | |
| Fw8 | 10 x 21d +CspA | 56 | 25 | 49 | 300,000 |
| 90096 | 10 x 21d +CspA | 31 | 20 | 15 | 3,000,000 |
| | | | | | |
| 99V | 10 x 21d +CspA+DBM | >475 | 53 | 84 | 20,000 |
| D7B | 10 x 21d +CspA+DBM | 65 | @65 | @65 | 1,000 |
| 90134 | 10 x 21d +CspA+DBM | 47 | @35 | 30 | 3,000,000 |
| AX | 10 x 21d +CspA+DMB | 33 | @33 | @33 | 10,000 |

| | | | | | |
|---|---|---|---|---|---|
| @ last sample tested at time of rejection | | | | | |

## Claims

1. Use of a humanized non-depleting anti-CD4 antibody and donor bone marrow in the manufacture of a medicament for administration to a primate subject for inhibiting rejection of a non-bone marrow donor allograft, wherein said donor bone marrow is from the allograft donor.

2. Use according to claim 1 wherein the humanized non-depleting anti-CD4 antibody is a CDR-grafted antibody.

3. Use according to claim 1 or claim 2, wherein the amount of antibody is sufficient to maintain lymphocyte CD4 saturation for a sufficient period to permit induction of immunological tolerance.

4. Use according to any one of claims 1 to 3, wherein the amount of antibody is from 0.5 to 10 mg/kg/day.

5. Use according to claim 4, wherein the amount of antibody is about 5 mg/kg/day.

## Patentansprüche

1. Verwendung eines humanisierten nicht-verringernden anti-CD4-Antikörpers und Spenderknochenmarks zur Herstellung eines Medikaments zur Verabreichung an ein Primatensubjekt zur Inhibierung der Abstoßung eines nicht-Knochenmark-Donor-Allografts, wobei das Spenderknochenmark von dem Allograft-Donor stammt.

2. Verwendung nach Anspruch 1, wobei der humanisierte nicht-verringernde anti-CD4-Antikörper ein CDR-gegrafteter Antikörper ist.

3. Verwendung nach Anspruch 1 oder Anspruch 2, wobei die Menge von Antikörper ausreichend ist, um eine CD4-Lymphozytensättigung für eine ausreichende Zeitdauer aufrechtzuerhalten, um eine Induktion von immunologischer Toleranz zu ermöglichen.

4. Verwendung nach einem der Ansprüche 1 bis 3, wobei die Menge von Antikörper von 0,5 bis 10 mg/kg/Tag ist.

5. Verwendung nach Anspruch 4, wobei die Menge von Antikörper ungefähr 5 mg/kg/Tag ist.

## Revendications

1. Utilisation d'un anticorps anti-CD4 humanisé ne provoquant pas de déplétion et de moelle osseuse de donneur dans la fabrication d'un médicament destiné à l'administration à un sujet primate pour inhiber le rejet d'une allogreffe qui n'est pas du donneur de moelle osseuse, dans laquelle ladite moelle osseuse de donneur est du donneur de l'allogreffe.

2. Utilisation selon la revendication 1 dans laquelle l'anticorps anti-CD4 humanisé ne provoquant pas de déplétion est un anticorps greffé à CDR.

3. Utilisation selon la revendication 1 ou la revendication 2, dans laquelle la quantité d'anticorps est suffisante pour maintenir la saturation en CD4 des lymphocytes pendant suffisamment longtemps pour permettre le déclenchement d'une tolérance immunologique.

4. Utilisation selon l'une quelconque des revendications 1 à 3, dans laquelle la quantité d'anticorps est de 0,5 à 10 mg/kg/jour.

5. Utilisation selon la revendication 4, dans laquelle la quantité d'anticorps est d'environ 5 mg/kg/jour.
